# EUROPEAN PATENT SPECIFICATION

(11) **EP 1 809 244 B1**
(45) Date of publication and mention of the grant of the patent: **12.05.2010**
(21) Application number: 05826137.1
(22) Date of filing: 09.11.2005
(51) Int. Cl.: A61K 9/12, A61K 47/08

(54) **RETINOID SOLUTIONS AND FORMULATIONS MADE THEREFROM**
RETINOIDLÖSUNGEN UND FORMULIERUNGEN DARAUS
SOLUTIONS DE RETINOIDES ET FORMULATIONS PREPAREES A PARTIR DE CES DERNIERES

(30) Priority: 09.11.2004 US 984630; 19.05.2005 US 132877
(43) Date of publication of application: 25.07.2007
(60) Divisional application: 09005071.7
(73) Proprietor: Imaginative Research Associates, Inc., Woburn MA 01801 (US)
(72) Inventor: VISHNUPAD, Mohan c/o Imaginative Research Associates, Inc., Woburn, Massachusetts 01801 (US); VISHNUPAD, Naomi, Reading, Massachusetts 01867 (US)
(74) Representative: Laufhütte, Dieter
(86) International application number: PCT/US2005/040510
(87) International publication number: WO 2006/053006

(56) References cited:
- WO-A-03/082703
- US-A- 5 690 923
- US-A1- 2002 082 745
- US-A1- 2003 215 493
- US-B1- 6 387 383

## Description

### Related Applications

The present application claims the benefit to and priority to U.S. Application Serial No. 11/132,877 filed on May 19, 2005 which is a continuation-in part of U.S. application Serial No. 10/984,630 filed November 9, 2004 which claims priority to U.S. Provisional application Serial No. 60/569,809 filed May 11, 2004. The entire disclosures of each of the above-related applications are incorporated herein by this reference.

### Background of Related Art

The combination of retinoids and antibiotics is of great interest in dermatology, due to the established synergistic efficacy of the two actives in treating acne. Retinoids are powerful keratolytic agents and antibiotics provide anti-bacterial activity for treating acne. Antibiotics such as erythromycin and clindamycin are soluble in aqueous media. Retinoids are insoluble in water. When formulating combination actives for treating acne, it is important to keep the retinoids in complete solution as well as antibiotics in solution. For example, aqueous retinoid acid formulations containing no alcohol and no fats have not shown to be clinically efficacious because the active ingredients are not dissolved in solution, and therefore not available for effectively treating the skin. See, U.S. Patent No. 5,690,923.

Unfortunately, retinoids alone in formulations have been known to be quite unstable. The stabilization of retinoids by dissolving in alcohols has been proposed. For example, U.S. Patent No. 5,721,275 discloses a topical composition containing a retinoid as a single active ingredient wherein in large concentrations of alcohol are used to dissolve the retinoids in solution. However, the stability of the composition containing high levels of alcohol is limited and high levels of alcohol will irritate the skin. Retinoids have also been formulated in aqueous vehicles using surfactants. For example, U.S. Patent No. 5,690,923 discloses the use of surfactants such as ethoxylated alcohol, ethers, and block polymers to solubilize retinoids in water without using any alcohol.

US-B1-6 387 383 shows an antibiotic i. e. clindamycin phosphate in combination with a retinoid, i. e. tretinoin lotion delivery system plus diisopropyl adipate a topical aqueous gel composition having a ph of 3-9 and a viscosity of < 15,000 cP for treating human skin disorder is described.

WO 03/082703 A describes a chamber-in-chamber dispenser with a main container, an auxiliary container, a discharge pump and a movable button portion to be used in food and pharmaceutical production.

In US-A-5 690 923 a composition is disclosed which comprises a liposomal vehicle, an antibiotic and a retinoid. The liposomal vehicle comprises a lipid phase disposed in an aqueous phase. The antibiotic is disposed in the aqueous phase. The retinoid is disposed in the lipid phase.

There exists room for improvement in the area of formulating, packaging, storing and dispensing compositions containing both retinoids and antibiotics to satisfactorily provide a full two year expiration date. Specifically, a need exists for a composition containing a retinoid and an antibiotic in complete solution, in which both active are chemically and physically stable.

### SUMMARY

Solutions of retinoic acid, tretinoin and other retinoids that are not completely soluble in alcohol are provided. The solutions include anhydrous alcohol in an amount insufficient alone to solubilize the retinoid and an ester co-solvent. The solutions can be used alone or to formulate topical compositions, such as emulsions or suspensions. In particularly useful embodiments, the formulations contain a water-soluble active in an aqueous phase and the retinoid solution in a non-aqueous phase.

In some embodiments, the present retinoid solutions are dispersed via a chamber in chamber pump delivery system.

The present compositions do not use any surfactants or emulsifiers to solubilize retinoids. Rather, the present retinoid solutions employ cosolvents, (such as alkyl benzoate, isopropyl palmitate ("IPP"), isopropylmyristate ("IPM"), diisopropyl adipate, or their derivatives) in conjunction with a small amount of alcohol. A benefit of the present solutions is that the amount of alcohol employed in the emulsion or suspension is so insignificant that the alcohol induced skin irritation is eliminated.

### BRIEF DESCRIPTION DF THE DRAWINGS

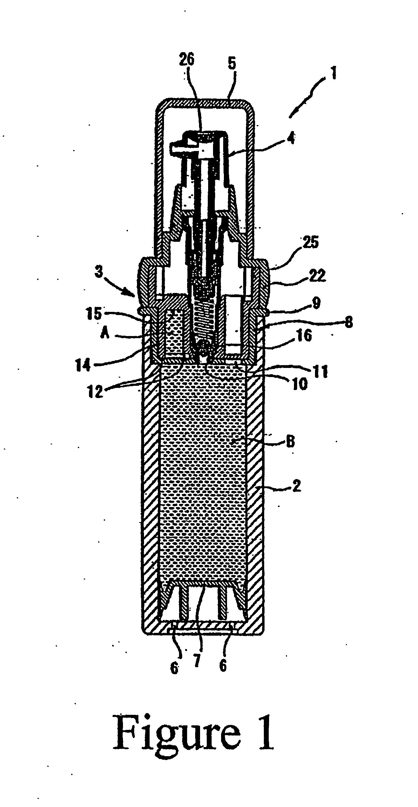

Figures 1-3 show one type of dispenser suitable for packaging and delivering formulations containing the present retinoid solutions.

### DETAILED DESCRIPTION OF PREFERRED EMBODIMENTS

Solutions of retinoids in accordance with this disclosure contain anhydrous alcohol in an amount insufficient alone to solubilize the retinoid, and an ester cho-solvent

The retinoid can be any retinoid that provides a benefit to a user upon topical application and is not completely soluble in alcohol. Suitable materials include, but are not limited to retinoic acid, retinyl palmitate, retinyl propionate, retinyl acetate, tretinoin, isotretinoin, motretinide, adapalene, tazarotene, azelaic acid as well as synthetic retinoid mimetics.

The anhydrous alcohol is present in an amount insufficient alone to solubilize the retinoid. At such low levels, the amount of alcohol present is less likely to be sufficient to irritate the skin of a user. Suitable anhydrous alcohols include anhydrous ethanol and anhydrous isopropanol.

The cosolvent can be any material which, when combined with the small amount of anhydrous alcohol, completely solubilizes the retinoid. Particularly useful cosolvents include esters, such as alkyl benzoates, isopropyl palmitate ("IPP"), isopropyl myristate ("IPM"), diisopropyl adipate and their derivatives. Long chain alkylbenzoates are one type of benzoic acid ester useful as a co-solvent in preparing the present retinoid solutions. The alkyl group of the alkyl benzoate preferably contains from 12 to 15 carbon atoms. Suitable alkyl benzoates are commercially available, for example, under the tradename FINSOLV (Finetex, Inc., Elmwood Park, N.J) such as FINSOLV-TN and FINSOLV-P (PPG-15 stearyl ether benzoate). Other suitable benzoate esters include Poloxamer 182 Dibenzoate, Poloxamer 105 benzoate and stearyl benzoate. Suitable benzoic acid esters are described for example in U.S. Pat. Nos. 4,275,222; 4,278,655; 4,293,544; 4,322,545; and 4,323,694.

While the amounts of each component of the present solutions will depend on the particular ingredients chosen, generally retinoid solutions in accordance with this disclosure may contain from 0.001 to 10 percent by weight retinoid, 0.003 to 40 percent by weight anhydrous alcohol and 50 to 99 percent by weight cosolvent.

The method of preparing the solution is not critical. Typically, the cosolvents are combined and the retinoid is added with stirring at room temperature until completely solubilized.

The retinoid solution can be used to formulate topical compositions, such as emulsions or suspensions. In particularly useful embodiments, the topical formulations contain a second active ingredient. The second active ingredient can be useful in treating acne, such as antibiotics (e.g., clindamycin, tetracyclone or erythromycin). In particularly preferred embodiments, retinoid solutions in accordance with this disclosure are included in the non-aqueous phase of an emulsion or suspension, the aqueous phase of which contains a water-soluble active that is incompatible with the retinoid. In certain embodiments, thermal stability of an emulsion or suspension formulation containing both a retinoid and clindamycin in combination is achieved using the present retinoid solutions.

The formulation of oil-in-water emulsions or powder suspensions using the present retinoid solutions is within the purview of one skilled in the art given the present disclosure. Typically, an emulsion or suspension is prepared, and a retinoid solution in accordance with this disclosure is added with adequate stirring to provide homogenous incorporation of the solution. Exemplary formulations are provided in the working examples, infra.

In order that those skilled in the art may be better able to practice the compositions and methods described herein in connection with the retinoid solution embodiments, the following examples are given as illustrations of the preparation of the present retinoid solutions and compositions containing them. It should be noted that the invention is not limited to the specific details embodied in the examples.

It has been experimentally determined that tretinoin is not soluble in ethanol in the ratios set forth in Examples A-D:

| Example | A | B | C | D |
|---|---|---|---|---|
| Tretinoin | 0.025 | 0.05 | 0.01 | 0.1 |
| Ethanol | 0.750 | 0.80 | 0.30 | 3.0 |

However, it has now been surprisingly found that when a cosolvent is added to compositions containing the same ratios of tretinoin to ethanol, the tretinoin will completely dissolve into a clear solution:

| Example | 1 | 2 | 3 | 4 |
|---|---|---|---|---|
| Tretinoin | 0.05 | 0.025 | 0.01 | 0.1 |
| Ethanol | 0.80 | 0.750 | 0.30 | 3.0 |
| IPM | 3.50 | 1.72 | 0.70 | 7.00 |

Examples 1-4 are clear yellow solutions of tretinoin. Isopropyl myristate ("IPM") can be replaced with alkyl benzoate, isopropyl palmitate ("IPP") or diisopropyl adipate to achieve the same results.

| Example | E | F | G | H | I | J |
|---|---|---|---|---|---|---|
| Tretinoin | 0.1 | 0.05 | 0.025 | 0.01 | 0.05 | 0.025 |
| Alkyl benzoate | 9.90 | - | - | 9.90 | - | - |
| IPM | - | 4.95 | - | - | - | - |
| IPM | - | - | 2.475 | - | - | - |
| Diisopropyl Adipate | - | - | - | - | 4.95 | 2.475 |

In Examples E-J where alcohol is not present, the retinoids are not soluble in the esters alone. This further confirms the present finding that retinoids can be solubilized in a small amount of alcohol by using specific cosolvents.

Further exemplary formulations made using retinoid solutions in accordance with this disclosure are presented in Examples 5-9:

| | Examples | | | | |
|---|---|---|---|---|---|
| Ingredient | 5 | 6 | 7 | 8 | 9 |
| Dionized water | QS | QS | QS | QS | QS |
| Disodium EDTA | 0.40 | 0.4 | 0.4 | 0.4 | 0.4 |
| Carbopol 980 | 0.20 | 0.2 | 0.2 | 0.2 | 0.2 |
| Steareth S-2 | 1.22 | 1.22 | 1.22 | 1.22 | 1.22 |
| Stearate (and) PEG-100 stearate | 0.896 | .896 | .896 | .896 | .896 |
| Cetyl stearyl alcohol | 1.22 | 1.22 | 1.22 | 1.22 | 1.22 |
| Emulsifier 10 | 0.80 | .80 | .80 | .80 | .80 |
| Glycerin | 13.79 | 13.79 | 13.79 | 13.79 | 13.79 |
| Butyl hydroxyl toluene | 0.05 | .05 | .05 | .05 | .05 |
| Sorbic acid | 0.10 | .10 | .10 | .10 | .10 |
| Clindamycin phosphate (100% active) | 1.00 | 1.00 | 1.00 | 1.00 | 1.00 |
| Tretinoic acid | 0.05 | 0.05 | 0.025 | 0.01 | 0.1 |
| Ethyl alcohol, anhydrous | 0.80 | 0.80 | 0.750 | 0.30 | 3.00 |
| IPM | 6.40 | 3.50 | 1.72 | 0.70 | 7.00 |

In examples 5-9 above, IPM can be replaced with alkyl benzoate (C₁₂-C₁₅ alkyl benzoate) or IPP.

### Examples 10 and 11: Emulsion Formulation

Active combination: clindamycin 1.00% w/w; tretinoin 0.05% w/w

| Ingredient | % Ex. 10 | % Ex. 11 |
|---|---|---|
| Dionized water | 71.5626 | 75.6824 |
| Disodium EDTA | 0.4 | 0.5 |
| NaOH (10%) | - | 0.35 |
| Carbopol 980 | 0.20 | 0.65 |
| Steareth S-2 | 1.216 | 1.9 |
| Stearate (and) PEG-100 stearate | 0.896 | 2.5 |
| Cetyl stearyl alcohol | 1.216 | 3.0 |
| Emulsifier 10 | 0.800 | 2.3 |
| Tween 20 | 0.80 | - |
| Fluilan | - | 0.36 |
| Glycerin | 13.79 | 1.9 |
| Butyl hydroxyl toluene | 0.040 | 0.05 |
| Stearaths-21 | - | 1.40 |
| Sorbic acid | 0.08 | 0.10 |
| Clindamycin phosphate (100% active) | 1.00 | 1.255 |
| Tretinoic acid | 0.0504 | 0.526 |
| Ethyl alcohol, anhydrous | 0.80 | 1.0 |
| Alkyl benzoate | 7.149 | - |
| IPM | - | 7.0 |

The emulsions of Example 10 and 11 is prepared as follows: Carbopol 980 is dispersed in water at 70-80 °C. Then, dissolve EDTA and mix well. The oil phase is prepared by combining steareth S-2, steareth S-21, tween 20 stearate and PEG-100 stearate, cetyl stearyl alcohol, emulsifier 10, Fluilan butyl hydroxy toluene and sorbic acid in the amounts indicated. The oil phase is heated to 75°C. Add the oil phase to the aqueous phase at 70-80°C with high shear mixing until a white emulsion is produced. Then cool the batch to room temperature. Dissolve the clindamycin in water and glycerin and add to the emulsion. Prepare a clear solution of tretinoin in alcohol and cosolvent. Add the tretinoin solution to the emulsion phase and continue mixing at high shear until uniform creamy emulsion is produced.

The elevated temperature stability of the actives in the oil in water emulsion of Example 10 was determined using techniques within the purview of those skilled in the art. The results were as follows:

| | | | |
|---|---|---|---|
| Clindamycin | | 25°C | 30°C |
| Initial | 0.975% | - | - |
| 1 month | | - | 0.958% |
| 2 months | | 0.975% | 0.958% |
| 3 months | | 0.975% | 0.958% |
| | | | |
| Tretinoin | | | |
| | | | |
| Initial | 0.0497% | - | - |
| 1 month | | - | 0.0490 |
| 2 months | | 0.0497 | 0.0490 |
| 3 months | | 0.0497 | 0.0490 |

### Examples 12 and 13 Liquid to Powder Suspension Systems

Liquid to powder suspension systems are prepared having the following compositions:

| Example | 12 | 13 |
|---|---|---|
| Ingredient | % | % |
| Clindamycin phosphate | 1.00 | 1.00 |
| Water | 15.8 | 15.15 |
| Glycerin | 13.80 | 13.80 |
| Propylene glycol | 14.50 | 14.50 |
| Volatile silicone | 35.0 | 35.00 |
| Modified starch | 15.0 | 15.00 |
| Tretinoin | 0.05 | 0.05 |
| Alcohol | 0.80 | 1.50 |
| Alkyl benzoate | 3.50 | 3.50 |
| BHT | 0.05 | - |
| Tween 20 | 0.50 | 0.50 |

Tretinoin is in a solution using a very small amount of alcohol and cosolvent, alkyl benzoate, IPM, IPP. Clindamycin will be in a clear solution in aqueous media with glycerin and propylene glycol. Both the clindamycin aqueous solution and the ester solution of tretinoin are suspended in the volatile silicone and starch. Both actives stay in one composition without interacting. Upon shaking, the composition delivers a therapeutic amount of the two actives for treating acne. Starch and volatile silicone provide excellent aesthetic vehicles, which upon application to the skin provide aesthetically acceptable liquid powder without any stickiness or tackiness. Furthermore, volatile silicone will evaporate from the skin surface, making the active easily available for acne treatment.

The liquid to powder suspension containing clindamycin and tretinoin is prepared as follows: Prepare a clear solution of tretinoin in alcohol and cosolvent. Prepare a clear solution of clindamycin in water and glycerin. Mix the volatile silicone and starch with a high shear mixer. Add polysorbate 20 and propylene glycol to the oil phase. Add clindamycin solution to the oil phase and continue mixing at high speed (shear). Add tretinoic solution to oil phase and continue mixing with a high shear mixer, until a smooth liquid to powder suspension is produced

The elevated temperature stability of the actives in the liquid to powder suspension system of Example 13 was determined using techniques known to those skilled in the art. The results were as follows:

| | | | |
|---|---|---|---|
| Clindamycin | | 25°C | 30°C |
| | | | |
| Initial | 1.00% | - | - |
| 1 month | | 0.999% | 0.991% |
| 2 months | | 0.990% | 0.977% |
| 3 months | | 1.02% | 1.01% |
| Tretinoin | | | |
| | | | |
| Initial | 0.0517% | - | - |
| 1 month | | 0.0508 | 0.051 |
| 2 months | | 0.0499 | 0.0498 |
| 3 months | | 0.0497 | 0.0488 |

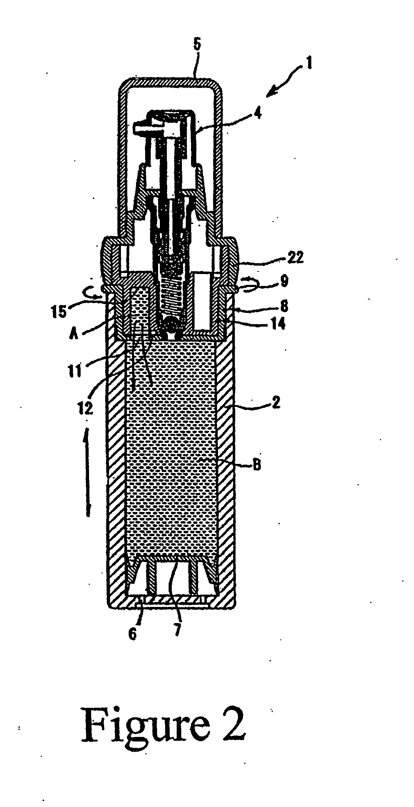

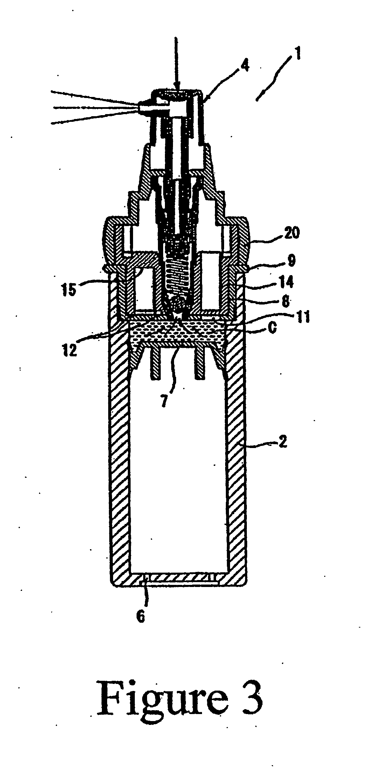

In another embodiment of this disclosure, by utilizing a chamber in a chamber single pump delivery system of the type disclosed in WO 03/082703A1 and EP1498362A1, the disclosures of which are incorporated herein in their entirety, the elevated temperature drug incompatibility is entirely overcome. One of the chambers may include a retinoid solution made in accordance with this disclosure using low levels of alcohol and one or more cosolvents. As seen in Figure 1, the small chamber 14, which has a small capacity, contains a composition (A) containing one of the active ingredients, such as antibiotics or retinoid. The composition in the small chamber could be either a solution or a powder blend. This chamber is inserted into a main chamber 2, which contains a composition (B) such as a thin lotion, suspension or emulsion, containing the other active ingredient. The small chamber is locked inside the main chamber. The two active drugs never come in contact with each other until the consumer activates the system before use. As shown in Figure 2, the consumer twists the main container to release the composition from the small chamber into the main chamber. The consumer then will shake the package to blend both products, which are specially formulated with low viscosity to facilitate quick and uniform blending. The consumer can then use the pump delivery mechanism (as shown in Figure 3) to dispense the blended-compositions to deliver the combination of both actives for treating the skin. The ratio of both actives is calculated for this system to deliver the combination of retinoid and antibiotics at a concentration that has already been established as acceptable by the FDA.

The shelf life or expiration date for such products, from the time of manufacturing to the time of patient's total consumption of the dispensed product, will be well over two years since the combined drugs are never exposed to elevated temperatures.
Furthermore, this system does not require any overage. The long shelf life and elimination of overage are big advantages from both the FDA perspective as well as the marketing viewpoint.

To effectively utilize the chamber in a chamber delivery system, one must balance the concentration of the actives in both chambers. This is necessary to achieve the final, active concentration, which is efficacious as well as compliant with the FDA. The present disclosure teaches how to prepare higher concentrations of tretinoin in solution for the small chamber by using lower concentrations of alcohol by means of cosolvents such a alkyl benzoate, isopropyl myristate, and isopropyl palmitate. For example, in accordance with the present methods 1 gram of tretinoin can be completely dissolved in only 3 grams of alcohol (33.3 percent solution) by using alkyl benzoate, isopropyl mystirate, isopropyl palmitate, or other esters as cosolvent.

In order that those skilled in the art may be better able to practice the compositions and methods described herein in connection with the chamber in chamber embodiment, the following examples are given as an illustration of the preparation of the present dispensing compositions and system. It should be noted that the invention is not limited to the specific details embodied in the examples.

### Example 14

The inner, smaller chamber of a chamber in a chamber package is filled with tretinoin solution composition and the larger, main chamber is filled with a clindamycin emulsion. The formulation for each composition is as follows:

| Small chamber composition | |
|---|---|
| Ingredient | % |
| Tretinoin 100% | 1.00 |
| Anhydrous alcohol | 30.00 |
| Alkyl benzoate | 69.00 |

### A. Composition in the main (large) chamber (Clindamycin Emulsion)

| Ingredient | | % |
|---|---|---|
| carbopol 980 | | 0.25 |
| glycerin 96% | | 2.00 |
| disodium EDT A | | 0.50 |
| deionized water | 78.92 | |
| Steareth S-21 | 1.12 | |
| Steareth S-2 | 1.52 | |
| Cetyl stearyl alcohol | 1.52 | |
| Arlacel 165 | | 1.52 |
| Emulsifier 10 | 1.84 | |
| Lanolin oil | 0.38 | |
| Tween 20 | | 1.75 |
| Alkyl benzoate ester | | 7.00 |
| Clindamycin PO₄ | | 1.28 |
| Sorbic acid | | 0.10 |
| Germall 115 | | 0.30 |
| | | |
| Ratio of composition A & B | | |
| Composition A | 5.00 | |
| Composition B | 95.00 | |

Once blended, the composition provides clindamycin at 1.0% and tretinoin at 0.05%

### Example 15

The inner, smaller chamber of a chamber in a chamber package is filled with tretinoin solution composition and the larger, main chamber is filled with a clindamycin suspension. The formulation for each composition is as follows:
A. Small chamber composition - tretinoin solution

| Ingredient | % |
|---|---|
| Tretinoin 100% | 1.00 |
| Anhydrous alcohol | 30.00 |
| Alkyl benzoate | 69.00 |

B. Main chamber composition - Clindamycin suspension

| Ingredient | % |
|---|---|
| Clindamycin PO₄ (82.7%) | 1.28 |
| H₂O | 16.60 |
| Glycerin 99% | 15.46 |
| Propylene glycol | 16.16 |
| Volatile silicone | 35.00 |
| Dry flo | 15.00 |
| Tween 20 | 0.50 |
| Ratio of composition A & B | |
| Composition A: | 5.00 |
| Composition B: | 95.00 |

Once blended, the composition provides Clindamycin at 1.00% and tretinoin at 0.05%.

Similar to the Tretinoin/Clindamycin combination, this system can also be used for Tretinoin and benzoyl peroxide, which is currently a non-marketed combination to treat acne. These 2 active ingredients are very unstable when combined together. The chamber in a chamber system permits the delivery of these actives together.

### Example 16

The inner, smaller chamber of a chamber in a chamber package is filled with tretinoin solution composition and the larger, main chamber is filled with a BPO suspension. The formulation for each composition is as follows:
A. Small chamber composition

| Ingredient | % |
|---|---|
| Tretinoin 100% | 1.00 |
| Anhydrous alcohol | 30.00 |
| Alkyl benzoate | 69.00 |

B. BPO Suspension in main chamber

| Ingredient | | % |
|---|---|---|
| BPO 75% | | 4.5 |
| Alkyl benzoate | | 10.00 |
| H₂O | | 15.00 |
| Glycerin | | 10.00 |
| Propylene glycol | | 10.00 |
| Volatile silicone | | 30.00 |
| Dry flo Starch | 20.00 | |
| Tween 20 | | 0.50 |
| | | |
| Blend: | Composition A: | 25 |
| | Composition B: | 75 |

The blend yields final concentration of 2.5% BPO and 0.025% tretinoin.

### Example 17

The inner, smaller chamber of a chamber in a chamber package is filled with tretinoin solution composition and the larger, main chamber is filled with a BPO emulsion. The formulation for each composition is as follows:
A. Small chamber composition

| Ingredient | % |
|---|---|
| Tretinoin 100% | 1.00 |
| Anhydrous alcohol | 30.00 |
| Alkyl benzoate | 69.00 |

B. Main Chamber: BPO Emulsion

| Ingredient | | % |
|---|---|---|
| Carbopol 980 | | 0.25 |
| Glycerin 96% | | 2.00 |
| EDTA | | 0.50 |
| Dionized water | 76.10 | |
| Brij 721 | | 1.12 |
| Brij 72 | | 1.52 |
| Cetyl stearyl alcohol | 1.52 | |
| Arlacel 165 | | 1.52 |
| Emulsifier 10 | | 1.84 |
| Lanolin oil | 0.38 | |
| Tween 20 | | 1.75 |
| Alkyl benzoate ester | | 7.00 |
| BPO 75% | | 4.5 |
| | | |
| Blend: | Composition A: | 25 |
| | Composition B: | 75 |

The blend yields final concentration of 2.5% BPO and 0.025% tretinoin.

Similarly, the small chamber can contain a benzoyl peroxide emulsion and the main chamber can contain a tretinoin solution, a tretinoin emulsion, or a tretinion suspension. The concentration of both the benzoyl peroxide and the tretinoin in the compositions is calculated in such a way the final blended ration of both compositions achieve the desired label claim requirement. Additionally, the clindamycin solution can be added to the tretinoin composition to provide a triple combination of benzoyl peroxide, tretinoin, and clindamycin either in a suspension of an emulsion.

### Example 18

### Composition A: Small Chamber: Benzoyl peroxide emulsion

| | |
|---|---|
| Dionized water | 64.31 |
| Disodium EDTA | 0.50 |
| Carbopol 980 | 0.25 |
| Steareth S-2 | 1.50 |
| Steareth S-21 | 1.10 |
| Stearate (and) PEG-100 stearate | 1.50 |
| Cetyl stearyl alcohol | 1.50 |
| Emulsifier 10 | 2.00 |
| Tween 20 | 2.00 |
| Glycerin | 2.00 |
| Alkyl benzoate | 10.00 |
| Benzoyl peroxide | 13.34 |

### Composition B: Tretinoin emulsion

| | |
|---|---|
| Dionized water | 82.67 |
| Disodium EDTA | 0.50 |
| Carbopol 980 | 0.25 |
| Steareth S-2 | 1.50 |
| Steareth S-21 | 1.10 |
| Stearate (and) PEG-100 stearate | 1.50 |
| Cetyl stearyl alcohol | 1.50 |
| Emulsifier 10 | 2.00 |
| Tween 20 | 2.00 |
| Glycerin | 2.00 |
| Sorbic acid | 0.10 |
| BHT | 0.05 |
| Tretinoin 100% | 0.033 |
| Alcohol anhydrous | 0.50 |
| Isopropyl myristate | 7.00 |
| | |
| Blend: Composition A: | 25 |
| Composition B: | 75 |

The blend of Composition A and B to yield 2.5% BPO and 0.025% tretinoin

Clindamycin phosphate can be added to the tretinoin emulsion above or the tretinoin suspension below to achieve a label claim of 1% in the final blend.

### Example 19

### Composition A: Small Chamber: Benzoyl peroxide emulsion

| | |
|---|---|
| Dionized water | 64.31 |
| Disodium EDTA | 0.50 |
| Carbopol 980 | 0.25 |
| Steareth S-2 | 1.50 |
| Steareth S-21 | 1.10 |
| Stearate (and) PEG-100 stearate | 1.50 |
| Cetyl stearyl alcohol | 1.50 |
| Emulsifier 10 | 2.00 |
| Tween 20 | 2.00 |
| Glycerin | 2.00 |
| Alkyl benzoate | 10.00 |
| Benzoyl peroxide | 13.34 |

### Composition B: Tretinoin suspension

| | |
|---|---|
| Tretinoin 100% | 0.033 |
| Anhydrous alcohol | 0.500 |
| Alkyl benzoate | 7.00 |
| H₂O | 10.00 |
| Glycerin | 10.00 |
| Propylene glycol | 10.00 |
| Volatile silicone | 41.967 |
| Dry flo starch | 20.00 |
| Polysorbate 80 (tween 20) | 0.50 |

It will be understood that various modifications may be made to the embodiments disclosed herein. For example, in embodiments where a tretinoin/ benzoyl peroxide combination is to be administered, the benzoyl peroxide composition can be an anhydrous composition, instead of an aqueous emulsion or suspension as illustrated in Examples 16 and 17, above. Suitable anhydrous benzoyl peroxide compositions are known and include but are not limited to the compositions described in U.S. Patent No. 5,632,996, the entire disclosure of which is incorporated herein by this reference. Therefore, the above description should not be construed as limiting, but merely as exemplifications of preferred embodiments. Those skilled in art will envision other modifications within the scope and spirit of the claims appended hereto.

## Claims

1. An apparatus comprising:
a first chamber containing a first composition, the first composition comprising a retinoid, an anhydrous alcohol and an ester, wherein the amount of anhydrous alcohol is insufficient alone to solubilize the retinoid;
a second chamber containing a second composition comprising benzoyl peroxide; means for selectively interconnecting the first and second chambers to allow mixing of the first and second compositions; and
at least one outlet for dispensing a mixture of the first and second compositions.

2. An apparatus as in claim 1 wherein the anhydrous alcohol is selected from the group consisting of anhydrous ethanol, anhydrous isopropanol and mixtures thereof.

3. An apparatus as in claim 1 wherein the ester is selected from the group consisting of alkyl benzoate, isopropyl palmitate, diisopropyl adipate, isopropyl myristate and mixtures thereof.

4. An apparatus as in claim 1 wherein the retinoid is tretinoic acid.

5. An apparatus as in claim 1 wherein the second composition is an aqueous suspension of benzoyl peroxide.

6. An apparatus as in claim 1 wherein the second composition is an aqueous emulsion of benzoyl peroxide.

7. An emulsion comprising:
an aqueous phase containing benzoyl peroxide; and
a non-aqueous phase containing a solution containing a retinoid, an anhydrous alcohol and an ester, wherein the amount of anhydrous alcohol is insufficient alone to solubilize the retinoid.

8. An emulsion as in claim 7 wherein the anhydrous alcohol is selected from the group consisting of anhydrous ethanol, anhydrous isopropanol and mixtures thereof.

9. An emulsion as in claim 7 wherein the ester is selected from the group consisting of alkyl benzoate, isopropyl palmitate, diisopropyl adipate, isopropyl myristate and mixtures thereof.

10. An emulsion as in claim 7 wherein the retinoid is tretinoic acid.

11. An emulsion as in claim 7 wherein the aqueous phase comprises an aqueous suspension of benzoyl peroxide.

12. An apparatus as in claim 7 wherein the aqueous phase comprises an aqueous emulsion of benzoyl peroxide.

13. A method comprising:
providing a first composition, the first composition comprising a retinoid, an anhydrous alcohol and an ester, wherein the amount of anhydrous alcohol is insufficient alone to solubilize the retinoid;
providing a second composition comprising benzoyl peroxide;
storing the first and second compositions separately from each other in first and second chambers, respectively of an apparatus comprising first and second chambers;
mixing the first and second compositions within the apparatus; and
dispensing the first and second compositions.

14. A method as in claim 13 wherein the anhydrous alcohol is selected from the group consisting of anhydrous ethanol, anhydrous isopropanol and mixtures thereof.

15. A method as in claim 13 wherein the ester is selected from the group consisting of alkyl benzoate, isopropyl palmitate, diisopropyl adipate, isopropyl myristate and mixtures thereof.

16. A method as in claim 13 wherein the retinoid is tretinoic acid.

17. A method as in claim 13 wherein the second composition comprises an aqueous suspension of benzoyl peroxide.

18. A method as in claim 13 wherein the second composition comprises an aqueous emulsion of benzoyl peroxide.

19. Apparatus according to claim 1 for the manufacture of a composition for administering to the skin of a subject.

20. Emulsion according to claim 7 for administering to the skin of a subject.

21. An apparatus as in claim 1 wherein the second composition is substantially anhydrous.

22. A method as in claim 13 wherein the second composition comprises substantially anhydrous benzoyl peroxide composition.

## Patentansprüche

1. Vorrichtung, welche umfasst:
eine erste Kammer, die eine erste Zusammensetzung enthält, wobei die erste Zusammensetzung ein Retinoid, einen wasserfreien Alkohol und einen Ester umfasst, wobei die Menge an wasserfreiem Alkohol allein ungenügend ist, um das Retinoid löslich zu machen;
eine zweite Kammer, die eine zweite Zusammensetzung enthält, welche Benzoylperoxid umfasst; Mittel zum selektiven Miteinanderverbinden der ersten und zweiten Kammer, um ein Mischen der ersten und zweiten Zusammensetzung zu ermöglichen; und
mindestens einen Auslass zum Abgeben einer Mischung der ersten und zweiten Zusammensetzung.

2. Vorrichtung nach Anspruch 1, **dadurch gekennzeichnet, dass** der wasserfreie Alkohol aus der Gruppe bestehend aus wasserfreiem Ethanol, wasserfreiem Isopropanol und Mischungen derselben gewählt ist.

3. Vorrichtung nach Anspruch 1, **dadurch gekennzeichnet, dass** der Ester aus der Gruppe bestehend aus Alkylbenzoat, Isopropylpalmitat, Diisopropyladipat, Isopropylmyristat und Mischungen derselben gewählt ist.

4. Vorrichtung nach Anspruch 1, **dadurch gekennzeichnet, dass** das Retinoid Tretinoinsäure ist.

5. Vorrichtung nach Anspruch 1, **dadurch gekennzeichnet, dass** die zweite Zusammensetzung eine wässrige Suspension von Benzoylperoxid ist.

6. Vorrichtung nach Anspruch 1, **dadurch gekennzeichnet, dass** die zweite Zusammensetzung eine wässrige Emulsion von Benzoylperoxid ist.

7. Emulsion, welche umfasst:
eine wässrige Phase, die Benzoylperoxid enthält; und
eine nichtwässrige Phase, die eine Lösung enthält, die ein Retinoid, einen wasserfreien Alkohol und einen Ester enthält, wobei die Menge an wasserfreiem Alkohol allein ungenügend ist, um das Retinoid löslich zu machen

8. Emulsion nach Anspruch 7, **dadurch gekennzeichnet, dass** der wasserfreie Alkohol aus der Gruppe bestehend aus wasserfreiem Ethanol, wasserfreiem Isopropanol und Mischungen derselben gewählt ist.

9. Emulsion nach Anspruch 7, **dadurch gekennzeichnet, dass** der Ester aus der Gruppe bestehend aus Alkylbenzoat, Isopropylpalmitat, Düsopropyladipat, Isopropylmyristat und Mischungen derselben gewählt ist.

10. Emulsion nach Anspruch 7, **dadurch gekennzeichnet, dass** das Retinoid Tretinoinsäure ist.

11. Emulsion nach Anspruch 7, **dadurch gekennzeichnet, dass** die wässrige Phase eine wässrige Suspension von Benzoylperoxid umfasst.

12. Vorrichtung nach Anspruch 7, **dadurch gekennzeichnet, dass** die wässrige Phase eine wässrige Emulsion von Benzoylperoxid umfasst.

13. Verfahren, welches umfasst:
Vorsehen einer ersten Zusammensetzung, wobei die erste Zusammensetzung ein Retinoid, einen wasserfreien Alkohol und einen Ester umfasst, wobei die Menge an wasserfreiem Alkohol allein ungenügend ist, um das Retinoid löslich zu machen;
Vorsehen einer zweiten Zusammensetzung, welche Benzoylperoxid umfasst;
Aufbewahren der ersten und zweiten Zusammensetzung separat voneinander in einer ersten bzw. zweiten Kammer einer Vorrichtung, welche eine erste und zweite Kammer umfasst;
Mischen der ersten und zweiten Zusammensetzung in der Vorrichtung; und
Abgeben der ersten und zweiten Zusammensetzung.

14. Verfahren nach Anspruch 13, **dadurch gekennzeichnet, dass** der wasserfreie Alkohol aus der Gruppe bestehend aus wasserfreiem Ethanol, wasserfreiem Isopropanol und Mischungen derselben gewählt ist.

15. Verfahren nach Anspruch 13, **dadurch gekennzeichnet, dass** der Ester aus der Gruppe bestehend aus Alkylbenzoat, Isopropylpalmitat, Diisopropyladipat, Isopropylmyristat und Mischungen derselben gewählt ist.

16. Verfahren nach Anspruch 13, **dadurch gekennzeichnet, dass** das Retinoid Tretinoinsäure ist.

17. Verfahren nach Anspruch 13, **dadurch gekennzeichnet, dass** die zweite Zusammensetzung eine wässrige Suspension von Benzoylperoxid umfasst.

18. Verfahren nach Anspruch 13, **dadurch gekennzeichnet, dass** die zweite Zusammensetzung eine wässrige Emulsion von Benzoylperoxid umfasst.

19. Vorrichtung nach Anspruch 1 für die Herstellung einer Zusammensetzung für das Verabreichen auf die Haut eines Probanden.

20. Emulsion nach Anspruch 7 für das Verabreichen auf die Haut eines Probanden.

21. Vorrichtung nach Anspruch 1, **dadurch gekennzeichnet, dass** die zweite Zusammensetzung im Wesentlichen wasserfrei ist.

22. Verfahren nach Anspruch 13, **dadurch gekennzeichnet, dass** die zweite Zusammensetzung im Wesentlichen wasserfreie Benzoylperoxidzusammensetzung umfasst.

## Revendications

1. Appareil comprenant:
une première chambre contenant une première composition, la première composition comprenant un rétinoïde, un alcool anhydre et un ester, où la quantité d'alcool anhydre est insuffisante seule pour solubiliser le rétinoïde;
une deuxième chambre contenant une deuxième composition comprenant du benzoyl peroxyde; un moyen pour interconnecter sélectivement les première et deuxième chambres pour permettre le mélange des première et deuxième compositions; et
au moins une sortie pour distribuer un mélange des première et deuxième compositions.

2. Appareil selon la revendication 1, dans lequel l'alcool anhydre est sélectionné dans le groupe consistant en éthanol anhydre, isopropanol anhydre et leurs mélanges.

3. Appareil selon la revendication 1, dans lequel l'ester est sélectionné dans le groupe consistant en alkyl benzoate, isopropyl palmitate, diisopropyle adipate, isopropyl myristate et leurs mélanges.

4. Appareil selon la revendication 1, dans lequel le rétinoïde est l'acide trétinoïque.

5. Appareil selon la revendication 1, dans lequel la deuxième composition est une suspension aqueuse de benzoyl peroxyde.

6. Appareil selon la revendication 1, dans lequel la deuxième composition est une émulsion aqueuse de benzoyl peroxyde.

7. Emulsion comprenant:
une phase aqueuse contenant du benzoyl peroxyde; et
une phase non aqueuse contenant une solution contenant un rétinoïde, un alcool anhydre et un ester, où la quantité d'alcool anhydre est insuffisante seule pour solubiliser le rétinoïde.

8. Emulsion selon la revendication 7, dans laquelle l'alcool anhydre est sélectionné dans le groupe consistant en éthanol anhydre, isopropanol anhydre et leurs mélanges.

9. Emulsion selon la revendication 7, dans laquelle l'ester est sélectionné dans le groupe consistant en alkyl benzoate, isopropyl palmitate, diisopropyl adipate, isopropyl myristate et leurs mélanges.

10. Emulsion selon la revendication 7, dans laquelle le rétinoïde est l'acide trétinoïque.

11. Emulsion selon la revendication 7, dans laquelle la phase aqueuse comprend une suspension aqueuse de benzoyl peroxyde.

12. Appareil selon la revendication 7, dans lequel la phase aqueuse comprend une émulsion aqueuse de benzoyl peroxyde.

13. Procédé comprenant:
réaliser une première composition, la première composition comprenant un rétinoïde, un alcool anhydre et un ester, où la quantité d'alcool anhydre est insuffisante seule pour solubiliser le rétinoïde;
réaliser une deuxième composition comprenant le benzoyl peroxyde;
stocker les première et deuxième compositions séparément l'une de l'autre dans des première et deuxième chambres, respectivement d'un appareil comprenant les première et deuxième chambres;
mélanger les première et deuxième compositions dans l'appareil; et
distribuer les première et deuxième compositions.

14. Procédé selon la revendication 13, dans lequel l'alcool anhydre est sélectionné dans le groupe consistant en éthanol anhydre, isopropanol anhydre et leurs mélanges.

15. Procédé selon la revendication 13, dans lequel l'ester est sélectionné dans le groupe consistant en alkyl benzoate, isopropyl palmitate, diisopropyl adipate, isopropyl myristate et leurs mélanges.

16. Procédé selon la revendication 13, dans lequel le rétinoïde est l'acide trétinoïque.

17. Procédé selon la revendication 13, dans lequel la deuxième composition comprend une suspension aqueuse de benzoyl peroxyde.

18. Procédé selon la revendication 13, dans lequel la deuxième composition comprend une émulsion aqueuse de benzoyl peroxyde.

19. Appareil selon la revendication 1, pour la fabrication d'une composition pour l'administration à la peau d'un sujet.

20. Emulsion selon la revendication 7 pour l'administration à la peau d'un sujet.

21. Appareil selon la revendication 1, dans lequel la deuxième composition est sensiblement anhydre.

22. Procédé selon la revendication 13, dans lequel la deuxième composition comprend une composition de benzoyl peroxyde sensiblement anhydre.
